# EUROPÄISCHE PATENTANMELDUNG

(11) **EP 2 360 136 A1**
(43) Veröffentlichungstag der Anmeldung: **24.08.2011**
(21) Anmeldenummer: 11165289.7
(22) Anmeldetag: 30.01.2003
(51) Int. Cl.: C07C 45/45, C07D 213/18

(54) **Hestellung von alkenonen**

(30) Priorität: 08.02.2002 DE 10205224; 31.12.2002 DE 10261471
(62) Teilanmeldung aus: 03704499.7
(71) Anmelder: Solvay Fluor GmbH, 30173 Hannover (DE)
(72) Erfinder: Braun, Max, Josef, 30900 Wedermark (DE); Claassen, Uta, 31249 Hohenhameln (DE)
(74) Vertreter: Mross, Stefan P.M.

(57) **Zusammenfassung**

Halogenalkenonether können durch die Anlagerung von Carbonsäurehalogeniden oder -anhydriden an einem Vinylether hergestellt werden. Die Verbesserung der vorliegenden Erfindung liegt darin, durch ein, zwei oder drei C1-C3-Alkylgruppen substituiertes Pyridin einzusetzen. Das Produkt fällt mit grosser Ausbeute an.

## Beschreibung

Die Erfindung bezieht sich auf ein Verfahren zur Herstellung von halogenierten Alkenonethern.

Halogenierte Alkenonether, beispielsweise 4-Ethoxy-1,1,1-trifluoro-3-buten-2-on, sind Bausteine in der chemischen Synthese, siehe beispielsweise EP-0 744 400. Man kann sie herstellen, indem man ein Säurechlorid mit einem Vinylether in Anwesenheit einer Base miteinander umsetzt, siehe die obengenannte europäische Offenlegungsschrift. Aufgabe der vorliegenden Erfindung ist es, ein verbessertes Verfahren anzugeben. Diese Aufgabe wird durch das Verfahren der vorliegenden Erfindung gelöst.

Das erfindungsgemäße Verfahren zur Herstellung von Alkenonen der Formel (I)

R¹-C(O)-C(H)=C(H)-OR² , (I)

wobei R¹ für eine C1-C4-Alkylgruppe oder für eine C1-C4-Alkylgruppe steht, die durch mindestens 1 Halogenatom substituiert ist, oder wobei R¹ für CF₃C(O) CH₂ steht, und wobei R² für Aryl, substituiertes Aryl, eine C1-C4-Alkylgruppe oder für eine C1-C4-Alkylgruppe steht, die durch mindestens 1 Halogenatom substituiert ist, sieht vor, daß man ein Säureanhydrid oder ein Säurehalogenid der Formel (II)

R¹-C (O) X , (II)

worin X für R¹-C(O)-O oder F, Cl oder Br steht und R¹ obengenannte Bedeutung besitzt, mit einem Vinylether der Formel (III)

CH = C(H)-OR² , (III)

worin R² die obengenannte Bedeutung besitzt, in Anwesenheit eines "Onium"-Salzes einer Carbonsäure miteinander umsetzt, oder wobei man durch 1 oder 2 C1-C3-Alkylgruppen substituiertes, ggf. chloriertes Pyridin einsetzt, oder wobei man ein "Onium"-Salz einer anorganischen Säure einsetzt.

Gemäß einer Variante der Erfindung kann durch 1, 2 oder 3 C1-C3-Alkylgruppen substituiertes Pyridin, vorzugsweise Picolin, Collidin oder Lutidin (d. h., Pyridin, das durch 1, 2 oder 3 Methylgruppen substituiert ist, dabei sind alle Isomere brauchbar), vorzugsweise 2-Picolin, eingesetzt werden. Das durch 1 bis 3 C1-C3-Alkylgruppen substituierte Pyridin kann auch im Kern und/oder der oder den Alkylgruppen durch ein oder mehr Chloratome substituiert sein. Dabei ist dann Chlormethyl-, Dichlormethyl- und Trichlormethylpyridin, insbesondere die in 2-Stellung substituierten Picoline, bevorzugt. Selbst wenn das gebildete Hydrochlorid verbrannt oder deponiert würde, ist diese Variante gegenüber anderen, im Stand der Technik verwendeten Aminen vorteilhaft wegen der höheren Ausbeute, die erzielt wird (es ist aber möglich, mittels Säurebehandlung ein Recycling vorzunehmen, wie weiter unten beschrieben wird).

Gemäß einer weiteren Variante setzt man ein "Onium"-Salz eines beliebigen Amins einer anorganischen Säure ein. Es wurde festgestellt, daß Addukte von Amin und Säure, auch anorganischer Säure, als Säurefänger bei der vorliegenden Erfindung wirksam sind, wenn das Mol-Verhältnis von Amin zur Säure kleiner als 3 ist. So ist beispielsweise Oniumhydrochlorid in der Lage, 2 Mol HCl, das aus der Reaktion stammt, abzufangen. Bei dieser Variante ist Oniumhydrochlorid bevorzugt.

Eine besonders bevorzugte Variante sieht die Verwendung von Onium-Carboxylaten beliebiger Amine vor. Dieses Verfahren besitzt den Vorteil einer milderen Reaktion und einer höheren Ausbeute, verglichen mit dem Verfahren des Standes der Technik, bei welchem ein Trialkylamin als Base verwendet wird, und wird im folgenden weiter erläutert.

R¹ steht bevorzugt für Methyl, Ethyl, n-Propyl oder i-Propyl oder durch mindestens 1 Fluoratom substituiertes Methyl, Ethyl, n-Propyl oder i-Propyl. Besonders bevorzugt steht R¹ für Methyl, Ethyl oder durch mindestens 1 Fluoratom substituiertes Methyl oder Ethyl. Ganz besonders bevorzugt steht R¹ für CF₃, CF₂H, CF₂Cl, C₂F₅, C₃F₇ oder CF₃C (O) CH₂ .

R² kann für Aryl, beispielsweise Phenyl oder C1-C4-Alkylgruppen und/oder Halogenatome substituiertes Phenyl stehen. Bevorzugt bedeutet R² lineares oder verzweigtes C1-C4-Alkyl. Ganz besonders bevorzugt bedeutet R² Methyl, Ethyl, n-Propyl oder i-Propyl.

Das Molverhältnis von "Onium"-Salz und Säurehalogenid oder Säureanhydrid liegt vorteilhaft zwischen 0,1:1 und 2:1.

Als Säurehalogenid ist das Säurechlorid bevorzugt. Anhand dieser bevorzugten Ausführungsform wird die Erfindung weiter erläutert.

Das Molverhältnis von Säurechlorid oder Anhydrid und Vinylether liegt zweckmäßig zwischen 0,9:1 und 1:0,8.

Die Umsetzung wird z. B. bei -15 bis +80 °C, vorteilhaft bei einer Temperatur im Bereich von ₀° bis 40 °C durchgeführt. Sie kann exotherm sein, so daß das Reaktionsgemisch gegebenenfalls gekühlt werden muß oder die Umsetzung sehr langsam durchgeführt wird.

Gemäß einer bevorzugten Ausführungsform verwendet man bei der Umsetzung ein Lösungsmittel. Dies ist besonders dann vorteilhaft, wenn man zum vorgelegten "Onium"-Salz oder Amin zunächst den Vinylether und dann das Anhydrid zufügt. Geeignet sind beispielsweise aliphatische lineare oder verzweigte Kohlenwasserstoffe oder aliphatische lineare oder verzweigte halogenierte Kohlen(wasser)stoffe, cyclische aliphatische Kohlenwasserstoffe oder Ester der Trifluoressigsäure oder der Pentafluorpropionsäure). Gut geeignet sind beispielsweise gegebenenfalls halogenierte Kohlen(wasser)stoffverbindungen mit 1 bis 8 C-Atomen. Sehr gut geeignet sind beispielsweise Dichlormethan, 1,1,1-Trifluor-2,2,2-trichlorethan, Hexan, Cyclohexan, Trifluoressigsäureethyl- oder propylester.

Gemäß einer anderen bevorzugten Ausführungsform verwendet man bei der Reaktion zwischen Anhydrid und Vinylether kein Lösungsmittel. Dies ist besonders dann gut möglich, wenn man zum vorgelegten "Onium"-Salz oder dem Amin zunächst das Anhydrid und dann den Vinylether zufügt. Vorteil ist, daß kein Lösungsmittel abgetrennt werden muß, was natürlich vorteilhaft ist (kein Aufwand für Rückgewinnung nötig, geringerer Energiebedarf).

Schließlich ist es auch möglich, die Umsetzung zwischen Vinylether und Säurehalogenid ohne Lösungsmittel durchzuführen, dann aber zur besseren Phasentrennung ein Lösungsmittel, z. B. CH₂Cl₂ zuzusetzen.

Das Anion der Carbonsäure des "Onium"-Salzes hat vorzugsweise die Formel R¹C(O)O-, wobei R¹ die obengenannte Bedeutung besitzt. Bei der Carbonsäure im verwendeten "Onium"-Salz der Carbonsäure handelt es sich vorzugsweise um diejenige Säure, die dem verwendeten Säurehalogenid entspricht.

Der Begriff "Onium" steht für Kationen mit positiv geladenem Stickstoff, beispielsweise protonierte aromatische Stickstoffbasen wie Pyridinium oder protonierte Alkyl-, Dialkyl- oder Trialkylammonium-Kationen oder für durch Cycloalkyl substituierte Ammonium-Verbindungen oder cycloaliphatische Stickstoffbasen wie Piperidinium oder quartäre Ammonium-Kationen.

Sehr gut geeignet als Carbonsäuresalz sind "Onium"-Salze, wobei "Onium" für ein Kation des Stickstoffs der Formel R'R"R"'R""N⁺ steht. R', R", R"' und R"" stehen unabhängig voneinander für Wasserstoff, Alkyl mit 1 bis 20 C-Atomen, Aryl oder Aralkyl. R' und R" oder R"' und R"", oder R', R" und R"' oder R', R", R"' und R"" können auch, gegebenenfalls unter Einschluß des Stickstoff-Atoms, gesättigte oder ungesättigte Ringsysteme bilden. "Aryl" bedeutet hier insbesondere Phenyl oder durch 1 oder mehrere C1-C2-Alkylgruppen substituiertes Phenyl. Hervorragend geeignet sind Salze, in denen "Onium" für Ammonium, Pyridinium oder R^{1'}R^{2'}R^{3'}R^{4'}N⁺ steht, worin R^{1'}, R^{2'}, R^{3'} und R^{4'} unabhängig voneinander Wasserstoff, Alkyl mit 1 bis 15 C-Atomen, Phenyl oder Benzyl stehen. Als Beispiel für solche Kationen seien genannt Pyridinium, Piperidinium, N-Methylpiperidinium, Anilinium, Benzyltriethylammonium und Triethylammonium.

Brauchbar sind auch durch Hydroxygruppen substituierte Amine, besonders cycloaliphatische Amine, insbesondere hydroxysubstituierte Piperidine und N-C1-C4-Alkylpiperidine. Geeignet sind z. B. die am C4-Atom substituierten Piperidine wie 4-Hydroxypiperidin, N-Methyl-4-hydroxypiperidin, N-Ethyl-4-hydroxypiperidin und N-Propyl-4-hydroxypiperidin.

Brauchbar sind auch Kationen von Aminen, welche in der deutschen Offenlegungsschrift 101 04 663.4 offenbart sind. Es handelt sich um "Onium"-Kationen auf Basis einer mono- oder bicyclischen Verbindung mit mindestens 2 Stickstoffatomen, wobei mindestens 1 Stickstoffatom in das Ringsystem eingebaut ist.

So kann man "Onium"-Kationen auf Basis von monocyclischen Verbindungen einsetzen. Es handelt sich dann um gesättigte oder ungesättigte 5-Ring-, 6-Ring- oder 7-Ring-Verbindungen. Mindestens 1 Stickstoffatom ist in den Ring eingebaut. Es kann auch noch ein weiteres Stickstoffatom in das Ringsystem eingebaut sein. Alternativ oder zusätzlich kann der Ring durch eine oder mehrere Aminogruppen substituiert sein. Bevorzugt sind Dialkylaminogruppen, in denen die Alkylgruppen gleich oder verschieden sein können und 1 bis 4 Kohlenstoffatome umfassen. Die Aminogruppe kann auch ein gesättigtes Ringsystem, beispielsweise eine Piperidinogruppe, darstellen. Gut brauchbare Vertreter von monocyclischen Ringsystemen sind Dialkylaminopyridin, Dialkylaminopiperidin und Dialkylaminopiperazin.

Auch "Onium"-Kationen bicyclischer Verbindungen kann man einsetzen. Auch hier können 1, 2 oder mehr Stickstoffatome in das Ringsystem integriert sein. Die Verbindungen können durch eine oder mehr Aminogruppen substituiert sein. Bevorzugt sind wieder Dialkylaminogruppen, wobei die Alkylgruppen gleich oder verschieden sein können und 1 bis 4 C-Atome umfassen oder zusammen mit dem Stickstoffatom ein gesättigtes Ringsystem bilden, wie beispielsweise die Piperidinyl-Gruppe.

Aus dem vorstehend gesagten wird klar, daß bei dieser Ausführungsform mindestens 2 Stickstoffatome in den brauchbaren Verbindungen basische Eigenschaften aufweisen müssen und, je nach Art der Bindungen, an 2 oder 3 Kohlenstoffatome gebunden sind.

Ganz besonders bevorzugt sind "Onium"-Salze der Carbonsäure mit bicyclischen Aminen, insbesondere 1,5-Diaza-bicyclo[4.3.0] non-5-en (DBN) und 1,8-Diazabicyclo [5.4.0]-undec-7-cen (DBU). Auch die "Onium"-Salze von aromatischen Aminen, besonders solche mit einer, zwei oder drei elektronenschiebenden Gruppen, wie C1-C3-Alkylgruppen, sind gut brauchbar, z. B. Salze des 2-Picolins. Salze des im Kern, z.B. in 4-Stellung und/oder den Alkylgruppen chlorierten Picolins, z. B. Trifluoressigsäureaddukte des 2-Chlormethyl-, 2-Dichlormethyl- und 2-Trichlormethyl-picolins, sind flüssig und können daher sogar wie ein Lösungsmittel wirken.

Die "Onium"-Salze der Carbonsäuren kann man durch einfache Umsetzung der entsprechenden Amine mit den freien Säuren herstellen.

Das erfindungsgemäße Verfahren zur Herstellung von Alkenonen der Formel (I) kann bei erhöhtem Druck oder auch bei Umgebungsdruck durchgeführt, werden. Es kann batchweise oder halbkontinuierlich durchgeführt werden.

Die Aufarbeitung der Reaktionsgemische erfolgt nach üblichen Methoden. Beispielsweise kann man das gewünschte Alkenon der Formel (I) nach Abtrennen des Lösungsmittels (sofern darin enthalten) aus dem Gemisch herausdestillieren. Eine andere Möglichkeit besteht darin, das Reaktionsgemisch mit Wasser zu versetzen und das Alkenon, nach Wasserabtrennung durch übliche Abtrennungsmittel wie Natriumsulfat, aus der organischen Phase zu isolieren.

Eine bevorzugte Ausführungsform nutzt die Aufarbeitung unter 2-Phasen-Bildung aus. Hierfür bieten sich zwei besonders vorteilhafte Varianten an. Eine Variante sieht die Aufarbeitung unter Zusatz von Wasser vor. Es bildet sich eine organische Phase, die das gewünschte Produkt sowie das verwendete organische Lösungsmittel enthält. Die wäßrige Phase enthält das verbrauchte "Onium"-Salz. Sofern man als eines der Edukte das Säureanhydrid eingesetzt hat, liegt das "Onium"-Salz weitgehend als "Onium"-Salz der dem Anhydrid entsprechenden Carbonsäure vor. Wenn man das "Onium"-Salz der Carbonsäure als Säurefänger eingesetzt hat, liegt in diesem Fall in der wäßrigen Phase ein Überschuß an Säure vor. Will man das "Onium"-Salz wieder als Säurefänger einsetzen, muß das Verhältnis von "Onium"-Kation zu Carbonsäuregehalt auf den bevorzugten Bereich von 0,9:1 bis 1:0,9 gebracht werden. Dies wird am einfachsten durch Zusatz von soviel Alkohol, z. B. von C1-C4-aliphatischen Alkoholen, bewirkt, daß die über den gewünschten Gehalt hinaus vorliegende Säure unter Veresterung abreagiert und durch Destillation zusammen mit dem vorhandenen Wasser abgetrennt werden kann.

Wurde z. B. das Säurechlorid als Edukt eingesetzt, liegt das "Onium"-Salz in der wäßrigen Phase weitgehend als Hydrochlorid bzw. als ein mit Chlorid angereicherter Onium-Komplex vor. Zur Aufarbeitung setzt man es mit der entsprechenden Carbonsäure, z. B. Trifluoressigsäure, bevorzugt im 5- bis 10-fachen molaren Überschuß um. Bei höherer Temperatur wird freigesetzte Salzsäure abgedampft. Da man bei dieser Regenerierung üblicherweise einen Überschuß der Carbonsäure einsetzt, liegt dann wieder ein "Onium"-Salz der Carbonsäure mit einem Überschuß an Säure vor, der sich zum Wiedereinsatz nicht gut eignet. Es wird dann, wie schon oben beschrieben, ein Alkohol zugegeben, der unter Esterbildung mit dem Säureüberschuß reagiert. Der Ester kann dann abdestilliert werden, wobei Wasser mit abdestilliert.

Eine andere Ausführungsform sieht vor, daß man ein organisches Lösungsmittel zusetzt, das die Bildung zweier Phasen bewirkt. Hierzu werden Lösemittel, die bewirken, daß das Reaktionsgemisch in homogener Phase vorliegt, zunächst entfernt. Dann wird ein Lösemittel oder Lösemittelgemisch zugesetzt, welches die Aufspaltung in zwei Phasen bewirkt. Als brauchbar haben sich beispielsweise erwiesen: Ether, insbesondere Dialkylether, besonders Diethylether; Ester der Trifluoressigsäure, beispielsweise Trifluoressigsäureisopropylester; aliphatische Kohlenwasserstoffe, beispielsweise Hexan; cyclische Kohlenwasserstoffe, beispielsweise Cyclohexan; halogenierte Kohlenstoffverbindungen, beispielsweise 1,1,2-Trichlor-1,2,2-trifluorethan (CFC-113) oder Dichlormethan. Durch einfaches Ausprobieren ist es dem Fachmann ein leichtes, weitere Lösungsmittel zu ermitteln, die ebenfalls eine Ausbildung zweier Phasen bewirken.

Eine Phase enthält das Lösemittel und das gebildete Alkenon, die andere Phase im Wesentlichen das Salz. Die Phase, die das Alkenon enthält, wird abgetrennt, das Lösemittel entfernt und das Alkenon kann dann in üblicher Weise gereinigt werden, beispielsweise durch Destillation, sofern das überhaupt notwendig ist, denn das Produkt fällt meist schon in sehr hoher Reinheit an. Es hat sich gezeigt, daß auch bei dieser Ausführungsform die Ausbeute und Reinheit des Produkts sehr hoch ist.

Statt der Reaktion mit einer Carbonsäure wie Trifluoressigsäure kann eine Regenerierung auch durch Zusatz des Anhydrids der Carbonsäure, z. B. durch Zusatz von Essigsäureanhydrid oder Trifluoressigsäureanhydrid, erfolgen, bevorzugt durch Zusatz des Anhydrids derjenigen Carbonsäure, die dem verwendeten Säurechlorid entspricht. Es bildet sich dann das Säurechlorid und das "Onium"-Salz der Carbonsäure, die dann weiter gemäß dem erfindungsgemäßen Verfahren mit Vinylethern umgesetzt werden können.

Das "Onium"-Salz der Carbonsäure kann vorab durch Umsetzung der freien Base mit der Carbonsäure hergestellt werden. Es kann auch während der Reaktion hergestellt werden, indem man kontinuierlich oder diskontinuierlich in das Reaktionsgemisch das Carbonsäureanhydrid derjenigen Carbonsäure einleitet, die dem Säurechlorid entspricht.

Eine Abwandlung des erfindungsgemäßen Verfahrens sieht vor, daß man in einer ersten Stufe ein Aldehyd oder Säurechlorid der allgemeinen Formel (II) und eine Vinylether der allgemeinen Formel (III) in Anwesenheit einer Amin-Base umsetzt, wie dies beispielsweise in der EP-A-0 744 400 beschrieben ist. Das anfallende Aminhydrochlorid wird dann bevorzugt wie oben beschrieben regeneriert und erneut, bei dieser Ausführungsform in einer zweiten Stufe, wieder im erfindungsgemäßen Verfahren eingesetzt.

Ein weiterer Gegenstand der Erfindung sind Addukte eines Carbonsäureanions der Formel R¹C(O)0⁻ mit einem protonierten Kation von Pyridin, das durch eine, zwei oder drei C1-C3-Alkylgruppen, vorzugsweise durch eine, zwei oder drei Methylgruppen, substituiert ist. Bevorzugt sind solche Addukte mit dem Anion der Trifluoressigsäure. Dabei können diese Addukte zusätzlich bis zu 1 Mol der freien Säure pro Mol "Onium"-Salz enthalten.

Das protonierte Kation des durch 1 bis 3 C1-C3-Alkylgruppen substituierten Pyridins kann auch chloriert sein, insbesondere in den Alkylgruppen. So kann es sich um 2-Chlormethyl-, 2-Dichlormethyl- und 2-Trichlormethylpyridinium handeln.

Besonders bevorzugt ist Picoliniumtrifluoracetat (n = 0) sowie seine Addukte mit Trifluoressigsäure, insbesondere der Formel A-Bₙ, worin A für Picoliniumtrifluoracetat, B für Trifluoressigsäure und n für 0<n≤2 steht.

Noch ein Gegenstand der Erfindung ist die Verwendung von Pyridin, das durch 1, 2 oder 3 C-1-C3-Alkylgruppen substituiert ist, als Säurefänger. 2-Alkylpyridin mit Alkyl = Methyl, Ethyl oder Propyl ist bevorzugt.

Die folgenden Beispiele sollen die Erfindung weiter erläutern, ohne sie in ihrem Umfang einzuschränken.

### Beispiele

Die Beispiele 1 bis 8 erläutern die Herstellung unter Verwendung von Trifluoracetylchlorid, die Beispiele 9 bis 12 die Herstellung unter Verwendung von Trifluoressigsäureanhydrid. Beispiel 13 erläutert die Regenerierung des verbrauchten "Onium"-Salzes mit Trifluoressigsäure.

In den Beispielen 1 bis 3 erfolgt eine wäßrige Aufarbeitung.

### Beispiel 1:

Herstellung von 4-Ethoxy-1,1,1-trifluor-3-buten-2-on (ETFBO) mit Pyridiniumtrifluoracetat

### Reaktion:

CF₃-COCl+CH₂=CH-O-CH₂-CH₃→CF₃-CO-CH=CH-O-CH₂-CH₃

| Ansatz: | | |
|---|---|---|
| Pyridin | 0,4 mol | 31,6 g |
| Trifluoressigsäure (TFA) | 0,4 mol | 45,6 g |
| Ethylvinylether | 0,3 mol | 21,6 g |
| Trifluoracetylchlorid (TFAC) | 0,3 mol | 39,6 g |
| Dichlormethan | | 180,0 g |

### Durchführung:

In einem 500 ml Dreihalskolben mit Trockeneiskühler wurde zuerst das Pyridiniumtrifluoracetat hergestellt. Dazu wurde Pyridin vorgelegt und unter Rühren TFA zugetropft. Damit die Mischung nicht zu heiß wurde (da Reaktion stark exotherm) wurde mit einem Wasserbad gekühlt. Anschließend wurde Dichlormethan und Ethylvinylether zugegeben und unter Rühren TFAC eingeleitet. Die Reaktionstemperatur wurde mittels Wasserbad auf Raumtemperatur gehalten. Der Ansatz wurde beim Einleiten von TFAC leicht gelblich. Anschließend wurde der Ansatz noch 2¾ h bei Raumtemperatur gerührt und dann eine GC Probe gezogen (Probe wurde hydrolysiert). Der Umsatz betrug 97,2 %, die Selektivität zu 4-Ethoxy-1,1,1-trifluor-3-buten-2-on (ETFBO) war quantitativ.

Zur Aufarbeitung wurde Wasser zugesetzt und die zwei sich bildenden Phasen getrennt. Das Dichlormethan wurde aus der organischen Phase abdestilliert und das zurückbleibende Produkt feindestilliert. Der wäßrigen Phase wurde Trifluoressigsäure zugesetzt und das Gemisch zum Austreiben der HCl unter Rückfluß gehalten. Dann wurde, dem Überschuß an eingesetzter Trifluoressigsäure entsprechend, Ethanol zugesetzt und der sich bildende Trifluoressigsäureester zusammen mit Ethanol und Wasser als Azeotrop abdestilliert. Das verbleibende "Onium"-Salz der Trifluoressigsäure wurde dann wieder in die ETFBO-Herstellung eingesetzt.

### Beispiel 2:

Trifluoracetylierung von Ethylvinylether mit dem Trifluoressigsäure(TFA)-Salz von 1,5-Diazabicyclo[4.3.0]-non-5-en (DBN) **(TFAC Unterschuß)**

### Reaktion:

CF₃-COCl+CH₂=CH-O-CH₂-CH₃ → CF₃-CO-CH=CH-O-CH₂-CH₃

| Ansatz: | | |
|---|---|---|
| DBN | 0,2 mol | 24,8 g |
| TFA | 0,2 mol | 22,8 g |
| Ethylvinylether | 0,2 mol | 14,2 g |
| TFAC | 0,18 mol | 23,8 g |
| Dichlormethan | | 120 g |

### Durchführung:

In einem 250 ml Dreihalskolben mit Trockeneiskühler wurde zuerst das DBNxTFA hergestellt. Dazu wurde DBN vorgelegt und unter Rühren TFA zugetropft. Damit die Mischung nicht zu heiß wurde (da Reaktion stark exotherm), wurde mit einem Wasserbad gekühlt, DBNxTFA wurde fest. Anschließend wurde Dichlormethan, Ethylvinylether zugegeben und unter Rühren TFAC eingeleitet. Die Reaktionstemperatur wurde mittels Wasserbad auf Raumtemperatur gehalten. Der Ansatz wurde beim Einleiten von TFAC gelb. Anschließend wurde der Ansatz noch 1 h bei Raumtemperatur gerührt und dann eine GC Probe gezogen (Probe wird hydrolysiert). Der Umsatz an EVE war quantitativ, die Selektivität zu ETFBO betrug 93,4 %.

Aufarbeitung wie in Beispiel 1.

### Beispiel 3:

Trifluoracetylierung von Ethylvinylether mit DBN x TFA **(TFAC äquimolar)**

### Reaktion:

CF₃-COCl+CH₂+CH-O-CH₂-CH₃ → CF₃-CO-CH=CH-O-CH₂-CH₃

| Ansatz: | | |
|---|---|---|
| DBN | 0,05 mol | 6,2 g |
| TFA | 0,05 mol | 5,7 g |
| Ethylvinylether | 0,05 mol | 3,6 g |
| TFAC | 0,05 mol | 6,6 g |
| Dichlormethan | | 30 g |

### Durchführung:

In einem 100 ml Dreihalskolben mit Trockeneiskühler wurde zuerst das DBNxTFA hergesellt. Dazu wurde Dichlormethan mit DBN vorgelegt und unter Rühren TFA zugetropft. Damit die Mischung nicht zu heiß wurde (da die Reaktion stark exotherm war), wurde mit einem Wasserbad gekühlt. Anschließend wurde Ethylvinylether zugegeben und unter Rühren TFAC eingeleitet. Die Reaktionstemperatur wurde mittels Wasserbad auf Raumtemperatur gehalten. Der Ansatz wurde beim Einleiten von TFAC gelb. Anschließend wurde der Ansatz noch 1½ h bei Raumtemperatur gerührt und dann eine GC Probe gezogen (Probe wird hydrolysiert). Der Umsatz an EVE war quantitativ, die Selektivität zu ETFBO lag bei 95 %.

Aufarbeitung wie in Beispiel 1.

### Beispiel 4:

Trifluoracetylierung von Ethylvinylether mit DBN x TFA unter 2-Phasen-Bildung

### Reaktion:

CF₃-COCl+CH₂=CH-O-CH₂-CH₃ → CF₃-CO-CH=CH-O-CH₂-CH₃ + HCl

ETFBO = 4-Ethoxy-1,1,1-Trifluoro-3-butene-2-on

| Ansatz: | | |
|---|---|---|
| DBN | 0,20 mol | 24,8 g |
| TFA | 0,20 mol | 22,8 g |
| Ethylvinylether | 0,15 mol | 10,8 g |
| TFAC | 0,15 mol | 19,8 g |
| Dichlormethan | | 90 g |

### Durchführung:

In einem 250 ml Dreihalskolben mit Trockeneiskühler wurde zuerst das DBN x TFA hergestellt. Dazu wurden MeCl₂ und DBN vorgelegt und unter Rühren TFA zugetropft. Damit die Mischung nicht zu heiß wurde (da Reaktion stark exotherm), wurde mit einem Wasserbad gekühlt. Anschließend wurde Ethylvinylether zugegeben und unter Rühren TFAC eingeleitet. Die Reaktionstemperatur wurde mittels Wasserbad auf Raumtemperatur gehalten. Der Ansatz wurde beim Einleiten von TFAC orange. Anschließend wurde der Ansatz noch 2 h bei Raumtemperatur gerührt und dann eine GC Probe gezogen (Probe wird hydrolysiert). Der Ethylvinylether hatte sich vollständig umgesetzt. Im Rotationsverdampfer wurde nun unter Vakuum das Lösungsmittel Dichlormethan abgezogen, und die restliche Lösung wurde in mehrere Teilvolumina aufgeteilt, welche durch Versetzen mit einem eine 2. Phase bildenden Lösungsmittel aufgearbeitet wurden.

Die Teilvolumina wurden mit gleichen Volumenanteilen folgender Lösungsmittel versetzt, worauf sich jeweils eine 2. Phase bildete:
Beispiel 4.1: Diethylether
Beispiel 4.2: Trifluoressigsäureisopropylester
Beispiel 4.3: Hexan
Beispiel 4.4: Cyclohexan
Beispiel 4.5: 1,1,2--Trichlor-1,2,2-Trifluorethan (113)

### Aufarbeitung:

In der organischen Phase befand sich hauptsächlich das gewünschte Produkt ETFBO; das verbrauchte Aminsalz befand sich quantitativ in der anderen Phase. Die ETFBO-Phase wurde abgetrennt und nun schonend am Rotationsverdampfer durch Abziehen des Solvens im Vakuum mit einer Reinheit >98 % isoliert.

### Beispiel 5:

Trifluoracetylierung von Ethylvinylether mit DBU x TFA

### Reaktion:

CF₃-COCl+CH₂=CH-O-CH₂-CH₃ → CF₃-CO-CH=CH-O-CH₂-CH₃ + HCl ETFBO

DBU = 1,5-Dizabicyclo[5.4.0]undec-5-en

| Ansatz: | | |
|---|---|---|
| DBU | 0,2 mol | 30,4 g |
| TFA | 0,2 mol | 22,8 g |
| Ethylvinylether | 0,15 mol | 10,8 g |
| TFAC | 0,15 mol | 19,8 g |
| Dichlormethan (MeCl₂) | | 90 g |

### Durchführung:

In einem 250 ml Dreihalskolben mit Trockeneiskühler wurde zuerst das DBU x TFA hergesellt. Dazu wurden MeCl₂ und DBU vorgelegt und unter Rühren TFA zugetropft. Damit die Mischung nicht zu heiß wurde (da Reaktion stark exotherm), wurde mit einem Wasserbad gekühlt. Anschließend wurde Ethylvinylether zugegeben und unter Rühren TFAC eingeleitet. Die Reaktionstemperatur wurde mittels Wasserbad auf Raumtemperatur gehalten. Der Ansatz wurde beim Einleiten von TFAC orange. Anschließend wurde der Ansatz noch 2 h bei Raumtemperatur gerührt und dann eine GC Probe gezogen (Probe wird hydrolysiert). Eine 2. Probe wurde am nächsten Morgen gezogen (Ansatz hatte sich dunkel verfärbt). Der Ethylvinylether hatte sich vollständig zu ETFBO umgesetzt. Die Isolierung erfolgte anhand der in Beispiel 4 beschriebenen 2. Phasenmethode.

### Beispiel 6:

Trifluoracetylierung von Ethylvinylether mit Pyridin x TFA

### Reaktion:

CF₃-COCl+CH₂=CH-O-CH₂-CH₃ → CF₃-CO-CH=CH-O-CH₂-CH₃ + HCl ETFBO

| Ansatz: | | |
|---|---|---|
| Pyridin | 0,4 mol | 31,6 g |
| TFA | 0,4 mol | 45,6 g |
| Ethylvinylether | 0,3 mol | 21,6 g |
| TFAC | 0,3 mol | 39,6 g |
| Dichlormethan | | 180 g |

### Durchführung:

In einem 500 ml Dreihalskolben mit Trockeneiskühler wurde zuerst das Pyridiniumtrifluoracetat hergestellt. Dazu wurde Pyridin vorgelegt und unter Rühren TFA zugetropft. Damit die Mischung nicht zu heiß wurde (da Reaktion stark exotherm), wurde mit einem Wasserbad gekühlt. Anschließend wurde Dichlormethan und Ethylvinylether zugegeben und unter Rühren TFAC eingeleitet. Die Reaktionstemperatur wurde mittels Wasserbad auf Raumtemperatur gehalten. Der Ansatz wurde beim Einleiten von TFAC leicht gelblich. Anschließend wurde der Ansatz noch 2¾ h bei Raumtemperatur gerührt und dann eine GC Probe gezogen (Probe wird hydrolysiert). Der Ethylvinylether hatte sich nahezu vollständig umgesetzt. Der Umsatz betrug 97,2 % zu 4-Ethoxy-1,1,1-trifluor-3-buten-2-on (ETFBO). Am Rotationsverdampfer wurde nun unter Vakuum MeCl₂ abgezogen und die restliche Lösung wurde wieder in Teilvolumina aufgeteilt und durch Versetzen mit einem eine 2. Phase bildenden Lösungsmittel extrahiert.

Die Teilvolumina bildeten mit gleichen Volumenanteilen folgender Lösungsmittel eine 2. Phase :

### Beispiel 6.1: Hexan

### Beispiel 6.2: Cyclohexan

### Beispiel 6.3: 1,1,2-Trichlor-1,2,2-Trifluorethan (113)

In dieser 2. Phase befand sich wiederum hauptsächlich das gewünschte Produkt ETFBO; das verbrauchte Amin befand sich quantitativ in der anderen Phase. Die ETFBO-Phase wurde nun abgetrennt und schonend am Rotationsverdampfer durch Abziehen des Solvens im Vakuum mit einer Reinheit >98 % isoliert.

Die in den Beispielen 4 bis 6 beschriebene Aufarbeitung mittels Bildung zweier Phasen führte zu besonders hohen Ausbeuten, dabei wurde eine thermische Belastung der Reaktionsmischung vermieden.

### Beispiel 7 :

Trifluoracetylierung von Ethylvinylether/Verwendung von Picolin

### Reaktion:

CF₃-COCl+CH₂=CH-O-CH₂-CH₃ → CF₃-CO-CH=CH-O-CH₂-CH₃-+HCl

| Ansatz: | | |
|---|---|---|
| 2-Picolin | 0,20 mol | 18,6 g |
| TFA | 0,20 mol | 22,8 g |
| Ethylvinylether | 0,15 mol | 10,8 g |
| TFAC | 0,15 mol | 19,8 g |
| Dichlormethan | | 90 g |

### Durchführung:

In einem 250 ml Dreihalskolben mit Trockeneiskühler wurde zuerst das Picolintrifluoracetat hergestellt. Dazu wurden Dichlormethan und 2-Picolin vorgelegt und unter Rühren TFA zugetropft. Damit die Mischung nicht zu heiß wurde (da Reaktion stark exotherm), wurde mit einem Wasserbad gekühlt. Anschlie-βend wurde Ethylvinylether zugegeben und unter Rühren TFAC eingeleitet. Die Reaktionstemperatur wurde mittels Wasserbad auf Raumtemperatur gehalten. Der Ansatz wurde beim Einleiten von TFAC gelb. Anschließend wurde der Ansatz noch 2½ h bei Raumtemperatur gerührt und dann eine GC Probe gezogen (Probe wurde hydrolysiert). Der Ethylvinylether hatte sich vollständig umgesetzt. Jetzt wurde der Ansatz auf 150 g Eiswasser gegeben, die organische Phase 2 x mit Wasser gewaschen und über einen Rotavapor destilliert.

Das Dichlormethan wurde bei 28 °C Wasserbadtemperatur und 300 mbar abgezogen. Das 4-Ethoxy-1,1,1-Trifluor-3-Buten-2-on destillierte bei 64 °C Wasserbadtemperatur und 13 mbar über. Gemäß Gaschromatogramm betrug die Reinheit 98,0 %.
Die Ausbeute an ETFBO lag bei 94,6 %.

### Beispiel 8:

Trifluoracetylierung von Ethylvinylether, 1. Stufe: freie Base, 2. Stufe: "Onium"-Trifluoracetat als Säurefänger

### Stufe 1:

| Ansatz 1. Stufe: | | |
|---|---|---|
| 2-Picolin | 0,05 mol | 4,66 g |
| Ethylvinylether | 0,15 mol | 10,8 g |
| TFAC | 0,15 mol | 19,8 g |
| Dichlormethan | | 90 g |

### Durchführung 1. Stufe:

In einem 250 ml Dreihalskolben mit Trockeneiskühler wurden 2-Picolin, Dichlormethan und Ethylvinylether vorgelegt und unter Rühren TFAC eingeleitet. Die Reaktionstemperatur wurde mittels Wasserbad auf Raumtemperatur gehalten. Der Ansatz wurde beim Einleiten von TFAC gelb. Nach 2½ h - der Ethylvinylether hatte sich vollständig umgesetzt - wurde der Ansatz auf 150 g Eiswasser gegeben, 2x mit Wasser gewaschen und dann die organische Phase über einen Rotavapor destilliert. Das Dichlormethan wurde bei 24 °C Wasserbadtemperatur und 300 mbar abgezogen. Das 4-Ethoxy-1,1,1-Trifluor-3-Buten-2-on destillierte bei 65 °C Wasserbadtemperatur und 15 mbar über. Gemäß Gaschromatogramm betrug die Reinheit 97,4 %.
Die Ausbeute ETFBO lag bei 76,2%.

Der verbleibende Rückstand bestand weitgehend aus Picolinhydrochlorid. Der Rückstand wurde mit Trifluoressigsäure versetzt, HCl ausgetrieben, Ethanol zugesetzt, um überschüssige Trifluoressigsäure zum Ester umzusetzen (s. auch Beispiel 12c), das gebildete Picoliniumtrifluoracetat wurde dann in die 2. Stufe eingesetzt.

### Stufe 2:

Verwendung des nach Stufe 1 erzeugten Picoliniumtrifluoracetats

Analog zu Beispiel 7, das Picolin und die Trifluoressigsäure wurden aber nicht getrennt eingesetzt, sondern in Form des vorstehend erhaltenen "Onium"-Salzes.

### Beispiel 9:

Trifluoracetylierung von Ethylvinylether/Verwendung von Trifluoressigsäureanhydrid (TFAH)

### Reaktion:

(CF₃-CO) 2O + CH₂=CH-O-CH₂-CH₃ → CF₃-CO-CH=CH-O-CH₂-CH₃ + CF₃COOH

| Ansatz: | | |
|---|---|---|
| 2-Picolin | 0,20 mol | 18,6 g |
| TFA | 0,20 mol | 22,8 g |
| Ethylvinylether | 0,15 mol | 10,8 g |
| TFAH | 0,15 mol | 31,5 g |
| Dichlormethan | | 90 g |

### Durchführung:

In einem 250 ml Dreihalskolben mit Wasserkühler wurde zuerst das Picolintrifluoracetat hergesellt. Dazu wurden Dichlormethan und 2-Picolin vorgelegt und unter Rühren TFA zugetropft. Damit die Mischung nicht zu heiß wurde (da Reaktion stark exotherm), wurde mit einem Wasserbad gekühlt. Anschlie-βend wurde Ethylvinylether zugegeben und unter Rühren TFAH zugetropft. Die Reaktionstemperatur wurde mittels Wasserbad auf Raumtemperatur gehalten. Der Ansatz wurde beim Zutropfen von TFAH gelb. Es wurde noch 1 h gerührt und dann eine GC Probe gezogen (Probe wurde hydrolysiert). Am nächsten Morgen wurde eine weitere Probe gezogen - der Ethylvinylether hatte sich vollständig umgesetzt - und der Ansatz dann auf 150 g Eiswasser gegeben. Die Organic wurde noch 2 x mit Wasser gewaschen und dann über einen Rotavapor destilliert.

Das Dichlormethan wurde bei 24 °C Wasserbadtemperatur und 300 mbar abgezogen. Das 4-Ethoxy-1,1,2-Trifluor-3-Buten-2-on destillierte bei 68 °C Wasserbadtemperatur und 18 mbar über. Gemäß Gaschromatogramm betrug die Reinheit 97,9 %.
Die Ausbeute an ETFBO lag bei 87,96 %.

### Beispiel 10:

Trifluoracetylierung von Ethylvinylether/Verwendung von Trifluoressigsäureanhydrid und Picolin

### Reaktion:

(CF_{3~}CO) ₂O + CH₂=CH-O-CH₂-CH₃ → CF₃-CO-CH=CH-O-CH₂-CH₃ + CF₃COOH

| Ansatz: | | |
|---|---|---|
| Pyridin | 0,20 mol | 15,8 g |
| TFA | 0,20 mol | 22,8 g |
| Ethylvinylether | 0,15 mol | 10,8 g |
| TFAH | 0,15 mol | 31,5 g |
| Dichlormethan | | 90 g |

### Durchführung:

In einem 250 ml Dreihalskolben mit Wasserkühler wurde zuerst das Pyridin-trifluoracetat hergesellt. Dazu wurden Dichlormethan und Pyridin vorgelegt und unter Rühren TFA zugetropft. Damit die Mischung nicht zu heiß wurde (da Reaktion stark exotherm), wurde mit einem Wasserbad gekühlt. Anschließend wurde Ethylvinylether zugegeben und unter Rühren TFAH zugetropft. Die Reaktionstemperatur wurde mittels Wasserbad auf Raumtemperatur gehalten. Es wurde noch 1 h gerührt und dann eine GC Probe gezogen (Probe wurde hydrolysiert). Am nächsten Morgen wurde eine weitere Probe gezogen - der Ethylvinylether hatte sich vollständig umgesetzt.
Die Ausbeute an ETFBO lag bei 85,0 %.

Zur Aufarbeitung wurde Wasser zugegeben, die sich bildende organische Phase wie oben beschrieben behandelt, indem das

Dichlormethan abdestilliert und das Produkt feindestilliert wurde. Der wäßrigen Phase wurde Ethanol zugesetzt und ein Ester/Wasser/Ethanol-Azeotrop abdestilliert.

### Beispiel 11:

Trifluoracetylierung von Ethylvinylether/Verwendung von DBN

### Reaktion:

(CF₃-CO)₂O+CH₂=CH-O-CH₂-CH₃ → CF₃-CO-CH=CH-O-CH₂-CH₃+CF₃-CO-OH

| Ansatz: | | |
|---|---|---|
| DBN | 0,20 mol | 24,8 g |
| TFA | 0,20 mol | 22,8 g |
| Ethylvinylether | 0,15 mol | 10,8 g |
| TFAH | 0,15 mol | 31,5 g |
| Dichlormethan | | 90,0 g |

### Durchführung:

In einem 250 ml Dreihalskolben mit Wasserkühler wurde zuerst das DBN x TFA hergesellt. Dazu wurden MeCl₂ und DBN vorgelegt und unter Rühren TFA zugetropft. Damit die Mischung nicht zu heiß wurde (da Reaktion stark exotherm), wurde mit einem Wasserbad gekühlt. Anschließend wurde Ethylvinylether zugegeben und unter Rühren TFAH zugetropft. Die Reaktionstemperatur wurde mittels Wasserbad auf Raumtemperatur gehalten. Der Ansatz färbte sich gelb. Anschließend wurde der Ansatz noch 1,5 h bei Raumtemperatur gerührt und dann eine GC Probe gezogen (Probe wurde hydrolysiert). Der Ethylvinylether hatte sich vollständig umgesetzt.

Im Rotationsverdampfer wurde unter Vakuum bei Raumtemperatur MeCl₂ abgezogen und die restliche Lösung nach Aufteilung in Teilvolumina mit verschiedenen 2 Phasen bildenden Lösungsmitteln extrahiert. Es wurden als 2-Phasenextraktionsmittel Hexan, Pentan, Cyclohexan und 113 eingesetzt.

Die gesamte isolierte Ausbeute an ETFBO betrug 91 %.

### Beispiel 12:

Aufarbeitung von "Onium"-Hydrochlorid

### Beispiel 12a:

Aufarbeitung von Pyridiniumhydrochlorid

### Reaktion:

Pyridinhydrochlorid + 10 TFA → Pyridintrifluoracetat + HCl

| Ansatz: | | |
|---|---|---|
| Pyridinhydrochlorid | 0,05 mol | 5,8 g |
| TFA | 0,50 mol | 75,0 g |

### Durchführung:

In einem 100 ml Dreihalskolben mit Wasserkühler wurden Pyridinhydrochlorid und TFA vorgelegt und am Rückfluß gekocht. Nach 5, 8 und 15 h wurden Cl⁻ Proben gezogen.

**C1⁻ Analysen**

| **Probe** | **Zeit h** | **% Cl** |
|---|---|---|
| 1 | 0 | 2,71 |
| 2 | 5 | 0,67 |
| 3 | 8 | 0,54 |
| 4 | 15 | 0,50 |

### Beispiel 12 b:

Aufarbeitung von Picoliniumhydrochlorid

### Reaktion:

Picolinhydrochlorid + 10 TFA → Picolintrifluoracetat + HCl

| Ansatz: | | |
|---|---|---|
| Picolinhydrochlorid | 0,16 mol | 20,6 g |
| TFA | 1,60 mol | 182,4 g |

### Durchführung:

In einem 250 ml Dreihalskolben mit Wasserkühler wurden Picolinhydrochlorid und TFA vorgelegt und am Rückfluß gekocht. Nach 1 h und 7 h wurden Cl⁻ Proben gezogen.

**Cl⁻ Analysen**

| **Probe** | **Zeit h** | **%Cl** |
|---|---|---|
| 1 | 0 | 2,40 |
| 2 | 1 | 0,26 |
| 3 | 7 | 0,027 |

Das Chlorid läßt sich im Vergleich zu Pyridin einfacher austauschen.

### Beispiel 12 c:

### Umsetzung mit Ethanol

Das Reaktionsprodukt aus Beispiel 12 a) wurde erhitzt und überschüssige Trifluoressigsäure abdestilliert, bis Picoliniumtrifluoracetat als Addukt mit weiterer Trifluoressigsäure vorlag; pro mol Picoliniumtrifluoracetat waren zwei mol Trifluoressigsäure (Amin x 3 TFA) im Rückstand vorhanden. Eine weitere Abtrennung von Trifluoressigsäure aus diesem Addukt war durch Destillation nicht möglich. Es wurde pro mol Essigsäure 1 mol Ethanol zugegeben. Nach Abdestillieren des gebildeten Trifluoressigsäureethylesters, wobei auch etwas nichtreagiertes Ethanol und vorhandenes Wasser übergingen, verblieb das Picoliniumtrifluoracetat, das dann wieder in die erfindungsgemäße Umsetzung eingebracht werden konnte.

### Beispiel 13:

Trifluoracetylierung von Ethylvinylether ohne zugesetztes Lösungsmittel

### Reaktion:

(CF₃-CO) ₂O + CH₂ = CH-O-CH₂-CH₃ → CF₃-CO-CH = CH-O-CH₂-CH₃ + CF₃COOH

| Ansatz: | | |
|---|---|---|
| 2-Picolin | 0,10 mol | 9,3 g |
| TFA | 0,10 mol | 11,4 g |
| Ethylvinylether | 0,15 mol | 10,8 g |
| TFAH | 0,25 mol | 31,5 g |

### Durchführung:

In einem 100 ml Dreihalskolben mit Wasserkühler wurde zuerst das Picolintrifluoracetat hergestellt, indem 2-Picolin vorgelegt und unter Rühren TFA zugetropft wurde. Damit die Mischung nicht zu heiß wurde (da Reaktion stark exotherm), wurde mit einem Eiswasserbad gekühlt. Anschließend wurde TFAH zugegeben und unter Rühren Ethylvinylether zugetropft (Reaktion stark exotherm). Die Reaktionstemperatur wurde mittels Eiswasserbad auf Raumtemperatur gehalten. Die Reaktionsmischung wurde schon bei der Zugabe von TFAH gelb. Es wurde noch eine Stunde gerührt und dann eine GC Probe gezogen (Probe wird hydrolysiert). Der Umsatz zu ETFBO lag bei 91,3 %.

### Beispiel 14:

Trifluoracetylierung in Abwesenheit von Lösungsmittel, Phasentrennung unter Lösungsmittelzusatz

### Durchführung:

Beispiel 13 wurde wiederholt. Die Umsetzung wurde ohne Lösungsmittel durchgeführt, zur noch besseren Phasentrennung wurde dann Dichlormethan zugegeben. Wiederum war ein hoher Umsatz zu ETFBO festzustellen.

## Patentansprüche

1. Verfahren zur Herstellung von Alkenonen der Formel (I)
R1-C(O)-C(H)=C(H)-OR2 (I)
wobei R¹ für eine Cl-C4-Alkylgruppe oder für eine C1-C4- Alkylgruppe steht, die durch mindestens ein Halogenatom substituiert ist, oder wobei R¹ für CF₃C(O)CH₂ steht und R² für Aryl, substituiertes Aryl, eine Cl-C4-Alkylgruppe oder für eine C1-C4-Alkylgruppe steht, die durch mindestens ein Halogenatom substituiert ist, wobei man ein Säureanhydrid oder Säurehalogenid der Formel (II)
R¹-C(O)X, (II)
worin X für R¹-C(O)-O- oder F, Cl, Br steht und R¹ die obengenannte Bedeutung besitzt, mit einem Vinylether der Formel (III)
CH = C(H)-OR2 (III)
worin R² die obengenannte Bedeutung besitzt wobei man durch 1 oder 2 C1-C3-Alkylgruppen substituiertes, gegebenenfalls chloriertes Pyridin einsetzt.

2. Verfahren nach Anspruch 1, **dadurch gekennzeichnet, daß** R¹ für Methyl, Ethyl oder Propyl oder durch mindestens 1 Fluoratom substituiertes Methyl, Ethyl oder Propyl steht.

3. Verfahren nach Anspruch 1, **dadurch gekennzeichnet, daß** R¹ für CF₃, CF₂H, CF₂C1, C₂F₅, C₃F₇ oder CF₃C(O)CH₂ steht.

4. Verfahren nach Anspruch 1, **dadurch gekennzeichnet, daß** R² für Methyl, Ethyl, n-Propyl oder iso-Propyl steht.

5. Verfahren nach Anspruch 1, **dadurch gekennzeichnet, daß** man die Umsetzung bei einer Temperatur im Bereich von -15°C bis +80°C, vorzugsweise 0°C* bis 40°C durchführt.

6. Verfahren nach Anspruch 1, **dadurch gekennzeichnet, daß** man das Reaktionsgemisch in zwei Phasen überführt, wobei eine Phase das Alkenon-Produkt enthält.

7. Addukte eines Carbonsäureanions der Formel R¹C(O)O⁻, worin R¹ die oben angegebene Bedeutung besitzt, mit einem protonierten Kation des Pyridins, das durch eine, zwei oder drei C1-C3-Alkylgruppen substituiert ist, das gegebenenfalls chloriert sein kann, wobei die Addukte zusätzlich 0 bis 2 Mol der freien Säure, die dem Carbonsäureanion entspricht, pro Mol des Addukts enthalten können.

8. Picoliniumtrifluoracetat der Formel A-Bₙ, worin A das Picoliniumtrifluoracetat ist, B die Trifluoressigsäure und 0≤n≤2.

9. Addukt nach Anspruch 8, welches Picoliniumtrifluoracetat ist (n = 0).

10. Verwendung von Pyridin, das durch eine, zwei oder drei C1-C3-Alkylgruppen substituiert ist, insbesondere von Picolin, Lutidin oder Collidin, als Säurefänger.
